# EUROPÄISCHE PATENTSCHRIFT

(11) **EP 2 373 246 B1**
(45) Veröffentlichungstag und Bekanntmachung des Hinweises auf die Patenterteilung: **13.07.2016**
(21) Anmeldenummer: 09798871.1
(22) Anmeldetag: 17.11.2009
(51) Int. Cl.: A61B 34/20

(54) **VERFAHREN ZUM ERZEUGEN VON BILDDATEN FÜR EINE DARSTELLUNG EINES INSTRUMENTES UND SYSTEM**
METHOD FOR PRODUCING IMAGE DATA FOR A DISPLAY OF AN INSTRUMENT AND SYSTEM
PROCÉDÉ DE PRODUCTION DE DONNÉES D'IMAGE POUR UNE REPRÉSENTATION D'UN INSTRUMENT, AINSI QUE SYSTÈME CORRESPONDANT

(30) Priorität: 17.11.2008 DE 102008057744
(43) Veröffentlichungstag der Anmeldung: 12.10.2011
(73) Patentinhaber: Charité Universitätsmedizin Berlin, 10117 Berlin (DE)
(72) Erfinder: MUCHA, Dirk, 10961 Berlin (DE); KOSMECKI, Bartosz, 12047 Berlin (DE); KRUEGER, Timo, 10437 Berlin (DE); MARTINEZ, Sc. Horacio, 81543 München (DE)
(74) Vertreter: Pfenning, Meinig & Partner mbB
(86) Internationale Anmeldenummer: PCT/DE2009/001610
(87) Internationale Veröffentlichungsnummer: WO 2010/054646

(56) Entgegenhaltungen:
- EP-A1- 1 380 266
- DE-U1- 20 115 254
- US-A1- 2006 122 497

## Beschreibung

Die Erfindung bezieht sich auf ein Verfahren zum Erzeugen von Bilddaten für eine Darstellung eines Instrumentes, insbesondere eines medizinischen Instrumentes, auf einer Anzeigevorrichtung. Ferner betrifft die Erfindung auf ein System zur Darstellung eines Instrumentes sowie ein Computerprogrammprodukt.

### Hintergrund der Erfindung

Beim Arbeiten mit einem Instrument in einem nicht direkt einsehbaren Bereich ist es von Bedeutung, die aktuelle Lage des Instrumentes erfassen und in geeigneter Weise darstellen zu können. Bekannt ist, ein Instrument mit elektromagnetischen Lagesensoren zu versehen und ferner einen Feldgenerator vorzusehen, so dass eine Steuerungseinheit, die mit dem Feldgenerator und dem oder den Lagesensoren verbunden ist, Lagedaten des Instrumentes zu erfassen und auf einer Anzeigevorrichtung zur Darstellung zu bringen.

Aus dem Aufsatz *"*Plausibility check for error compensation in electromagnetic navigation in endoscopic sinus surgery" von Mucha et al., veröffentlicht als Proc. Computer Assisted Radiology and Surgery CARS, P. 316-18, 2006, ist bekannt, ein Instrument mit zwei Lagesensoren zu versehen. Zur Behandlung des Problems einer Feldstörung durch metallische Geräte ist eine Plausibilitätsprüfung von gemessenen Lagemessdaten vorgesehen. Es besteht jedoch das Problem, zusätzlich Fehler im Zusammenhang mit Bewegungen des Instrumentes in geeigneter Weise zu behandeln.

Im Dokument DE 10 2004 042 489 A1 ist eine medizinische Untersuchungs- und Behandlungseinrichtung offenbart.

Das Dokument EP 1 380 266 A1 offenbart ein System zum Überwachen der Lage eines medizinischen Instruments. Abhängig von der Lage des Instruments werden zuvor aufgenommene Bilder des Körpers eines Patienten auf einer Anzeigeeinrichtung dargestellt. An dem medizinischen Instrument sind mehrere Lagesensoren angeordnet, mit denen die Lage und eine Lageänderung des Instruments erfasst werden.

### Zusammenfassung der Erfindung

Aufgabe der Erfindung ist es, verbesserte Technologien zum Erzeugen von Bilddaten für eine Darstellung eines Instrumentes auf einer Anzeigevorrichtung zu schaffen, wobei insbesondere die Kontinuität der Anzeige und die Zuverlässigkeit der Anzeige verbessert werden sollen.

Diese Aufgabe wird erfindungsgemäß durch ein Verfahren zum Erzeugen von Bilddaten für eine Darstellung eines Instrumentes nach dem unabhängigen Anspruch 1 gelöst. Weiterhin sind eine System zur Darstellung eines Instrumentes nach dem unabhängigen Anspruch 11 sowie ein Computerprogramm-Produkt nach den unabhängigen Anspruch 14 geschaffen. Vorteilhafte Ausgestaltungen der Erfindung sind Gegenstand von abhängigen Unteransprüchen.

Als Lage eines Werkzeuges wird im Folgenden wenigstens der Ort des Instrumentes in Bezug auf ein bestimmtes Koordinatensystem und / oder wenigstens eine Orientierung des Instrumentes in diesem Koordinatensystem bezeichnet. Als Lagemessdaten werden im Folgenden insbesondere Feldstärkedaten von Lagesensoren bezeichnet, die dazu geeignet sind, wenigstens eine Position und/oder wenigstens eine Orientierung des Lagesensors und des Instrumentes, an welchem der Sensor befestigt ist, bereitzustellen. Ein Lagesensor kann beispielsweise als elektromagnetischer Sensor ausgeführt sein, beispielsweise als eine Spule oder ein Satz von Spulen, aber andere Einrichtungen, die dazu in der Lage sind, eine Orts- und / oder Lageinformation auszugeben sind auch möglich. Mit anderen Worten ist ein Verfahren bereitgestellt, mittels dessen ein Instrument auf einer Anzeigevorrichtung gezeigt werden kann, ohne dass ein Benutzer des Instrumentes einen direkten Sichtkontakt zu dem Instrument haben muss, und wobei die Darstellung des Instruments wenigstens auf der Grundlage von gefilterten Lagemessdaten vorgenommen wird, die aufgrund eines Bewegungszustands gefiltert wurden. Eine bevorzugte Weiterbildung der Erfindung sieht vor, dass die erfassten Lagemessdaten auf Grundlage wenigstens einer aktuellen Lagemessung mit aktuell erfassten Lagemessdaten gebildet werden. Somit liegen die aktuelle Lage des Instruments repräsentierende Daten vor, beispielsweise in einer Steuerung, und können verarbeitet werden.

Bei einer zweckmäßigen Ausgestaltung der Erfindung kann vorgesehen sein, dass die Lagemessdaten mittels mehrerer Lagemessungen erfasst werden. Dies wird beispielsweise mittels eines Speichers ermöglicht, der in einer Steuerungsvorrichtung vorgesehen ist, welche dazu hergerichtet ist, das Verfahren auszuführen. Die Mehrzahl von Lagemessungen kann beispielsweise innerhalb konstanter Zeitintervalle aufgenommen sein, so dass eine Messreihe von Lagemessungen vorliegt.

Die Erfindung sieht vor, dass auf Grundlage der erfassten Lagemessdaten ein Plausibilitätswert gebildet wird. Es sind wenigstens zwei Lagesensoren vorgesehen. Jeder der an dem Instrument angeordneten Lagesensoren ist konfiguriert, Lagemessdaten auszugeben, aus denen eine Lage des Instrumentes bestimmt werden kann. Insbesondere kann mittels geeigneter Transformationen ein Instrumentenarbeitspunkt bestimmt werden. Idealerweise führen die Auswertungen der Lagemessdaten aller an dem Instrument angeordneten Lagesensoren zu demselben Instrumentenarbeitspunkt. Befindet sich allerdings ein leitender, beispielsweise metallischer, Gegenstand in der Nähe eines der Lagesensoren, wird die Feldstruktur in der Nähe dieses Lagesensors verändert, so dass bei der Auswertung der Lagemessdaten dieses Lagesensors ein anderer Instrumentenarbeitspunkt ermittelt wird als bei der Auswertung der Lagemessdaten des weitestgehend ungestörten Lagesensors. Der Betrag des Abstandes zwischen den beiden ermittelten Instrumentenarbeitspunkten wird als Plausibilitätswert bezeichnet. Ein großer Plausibilitätswert bedeutet also eine große Abweichung der bestimmten Instrumentenarbeitspunkte. Ein auf diese Weise verursachter Fehler wird als Fremdstörung bezeichnet.

Bei einer vorteilhaften Ausgestaltung der Erfindung kann vorgesehen sein, dass der Bewegungszustand des Instruments auf der Grundlage der aktuell erfassten Lagemessdaten und in der Vergangenheit erfasster Lagemessdaten bestimmt wird. Unterschiedliche Bewegungen des Instrumentes wie beispielsweise Translationen oder Rotationen oder Kombinationen dieser Bewegungen, die mit großer oder kleiner Geschwindigkeit ausgeführt werden, können auf diese Weise ausgewertet und kategorisiert und schließlich einem Bewegungszustand zugeordnet werden.

Bei einer zweckmäßigen Ausgestaltung der Erfindung kann vorgesehen sein, dass jedem Bewegungszustand ein Plausibilitätsschwellwert zugeordnet wird. So ist es möglich, auf der Grundlage vergangener Bewegungen des Instrumentes einen Plausibilitätsschwellwert für eine weitere Auswertung festzulegen.

Bevorzugt sieht eine Fortbildung der Erfindung vor, dass das Filtern der erfassten Lagemessdaten einen Schritt zum Vergleichen des Plausibilitätswertes und des Plausibilitätsschwellwertes umfasst. Da der Plausibilitätswert von einer Feldstörung durch einen leitfähigen, beispielsweise metallischen, Körper in der Nähe eines der Lagesensoren beeinflusst und der Plausibilitätsschwellwert auf der Grundlage des Bewegungszustandes des Instrumentes bestimmt werden, wird eine Bewegung des Instrumentes in einem gestörten Feld betrachtet, so dass diese unterschiedlichen Fehlerquellen letztendlich mit in die Darstellung des Instrumentes auf der Anzeigevorrichtung einfließen können.

Bevorzugt sieht eine Fortbildung der Erfindung vor, dass das Filtern der erfassten Lagemessdaten einen Schritt zum Akzeptieren der erfassten Lagemessdaten für den Fall, dass der Plausibilitätswert kleiner als der oder gleich dem Plausibilitätsschwellwert ist, und einen Schritt zum Verwerfen der erfassten Lagemessdaten für den Fall umfasst, dass der Plausibilitätswert größer als der Plausibilitätsschwellwert ist. Ist also ein Plausibilitätswert größer als ein Plausibilitätsschwellwert, wird die entsprechende aktuelle Lage verworfen und nicht zur Anzeige gebracht. Auf diese Weise werden für einen Benutzer verwirrende Darstellungen oder Darstellungsänderungen vermieden.

Es ist vorgesehen, dass der Bilddatensatz wenigstens auf Grundlage der aktuell erfassten Lagemessdaten und des Plausibilitätswertes gebildet wird. Auf der Grundlage der aktuellen Lagemessdaten wird der Instrumentenarbeitspunkt ermittelt, und der Plausibilitätswert als Radius einer um den Instrumentenarbeitspunkt beschriebenen Kugel ausgewertet.

Auf diese Weise wird einem Benutzer eine Unsicherheit des aktuellen Instrumentenarbeitspunktes visualisiert.

Bei einer zweckmäßigen Ausgestaltung der Erfindung kann vorgesehen sein, dass auf der Grundlage der erfassten Lagemessdaten und des Plausibilitätswertes bestimmt wird, ob eine Eigenstörung und / oder eine Fremdstörung vorliegt.

Bevorzugt sieht eine Fortbildung der Erfindung vor, dass die Fremdstörung auf der Grundlage des Plausibilitätswertes und die Eigenstörung auf der Grundlage der erfassten Lagemessdaten bestimmt werden. Als Fremdstörung wird insbesondere eine Störung angesehen, die durch einen leitfähigen, beispielsweise metallischen, Gegenstand zu Lagemessdatensätzen der Lagesensoren führt, auf deren Grundlage abweichende Instrumentenarbeitspunkte ermittelt werden. Als Eigenstörung wird eine Bewegung, beispielsweise ein Positionieren, des Instrumentes angesehen. Das bedeutet, dass die gemessene Instrumentenposition nicht oder durch unzureichende Kalibrierdaten beeinflusst wird. Eine Eigenstörung kann auch ein Rotieren des Instrumentes sein. Das bedeutet, dass die gemessene Instrumentenposition durch die Rotationsbewegung beeinflusst wird. Eine Eigenstörung kann ferner auch ein Bewegen des Instrumentes umfassen. Das bedeutet, dass die gemessene Instrumentenposition durch die Translationsbewegung beeinflusst wird.

Das erfindungsgemäße Verfahren sowie das System und das Computerprogrammprodukt ermöglichen in der oben geschilderten Weise einem Benutzer eines Instrumentes eine sichere Instrumentenführung, sogar, wenn er keinen Sichtkontakt zu dem Instrument hat. Das Instrument kann beispielsweise ein Bohrer, Führungsdraht, Katheter, Endoskop, Biopsienadel, Schneidinstrument oder jegliches andere Instrument, insbesondere auf dem medizinischen Gebiet, sein.

Weiterhin ist ein wenigstens ein Navigationsmodul und einen Monitor umfassendes Endoskopiesystem bereitgestellt, das dazu konfiguriert ist, wenigstens eine synchronisierte Video- und Navigationsinformation auf dem Monitor, insbesondere Endoskopiemonitor, bereitzustellen. Vorzugsweise ist das Endoskopiesystem dazu konfiguriert, eine auf der Grundlage des oben beschriebenen Verfahrens synchronisierte Video- und Navigationsinformation auf dem Monitor darzustellen. Die Synchronisation umfasst beispielsweise eine Aktualisierung der, wie oben beschrieben, um den Instrumentenarbeitspunkt herum dargestellten, auf der Grundlage des Plausibilitätswertes und der erfassten Lagemessdaten bestimmten Kugel zur Veranschaulichung einer Messunsicherheit. Die Aktualisierung der Darstellung kann beispielsweise auf einer Aktualisierung des jeweils zur Anzeige gebrachten Bilddatensatzes beruhen.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Darstellung eines Instrumentes mit zwei Lagesensoren und Koordinatensystemen,
- Fig. 2: ein Instrument wie in Fig. 1 mit einem Störkörper,
- Fig. 3: ein Konzept der adaptiven Schwellwertfilterung,
- Fig. 4: ein Konzept einer adaptiven Schwellwertfilterung wie in Fig. 3 mit einer Erweiterung,
- Fig. 5: einen Klassifizierungsalgorithmus zum Bestimmen eines Bewegungszustandes des Instrumentes,
- Fig. 6a: ein Beispiel einer Visualisierung der Instrumentenspitze mit Fehlerkugel an einem Patienten
- Fig. 6b: die Ansicht von Fig. 6a in einem Draufsichtschnitt,
- Fig. 7: einen schematischen Aufbau eines Endoskopiesystems mit Endoskopiemonitor und
- Fig. 8: einen Endoskopiemonitor gemäß Fig. 7.

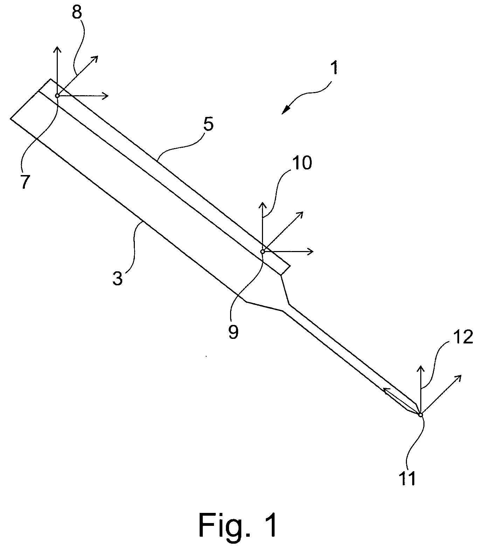

Fig. 1 zeigt ein Instrument 1, welches in der dargestellten Ausführungsform beispielhaft als ein medizinisches Instrument gebildet ist und an dem ein Lokalisator 5 fest angeordnet ist, an welchem zwei Lagesensoren 7 und 9 angebracht sind. Das Instrument 1 ist mit einer Spitze 11 versehen, die den Instrumentenarbeitspunkt darstellt. Den relevanten Punkten sind lokale Koordinatensysteme zugeordnet: Dem Lagesensor 7 ist ein Koordinatensystem 8 mit Ursprung 7 zugeordnet, dem Lagesensor 9 ist ein Koordinatensystem 10 mit Ursprung 9 zugeordnet und dem Instrumentenarbeitspunkt 11 ist ein Koordinatensystem 12 mit Ursprung 11 zugeordnet. Die Lagesensoren 7 und 9 erfassen ein von einer oder mehreren Feldspulen erzeugtes elektromagnetisches Feld. Die Messwerte der Lagesensoren 7 und 9 werden an eine Steuereinrichtung (hier nicht gezeigt) weitergeleitet. Die Steuereinrichtung ist konfiguriert, aus den Lagemessdaten des jeweiligen Lagesensors 7 und 9 eine Lage der lokalen Koordinatensysteme 8 und 10 zu berechnen. Die Lage betrifft hier insbesondere den Ort in einem dreidimensionalen Koordinatensystem wie auch die Orientierung des jeweiligen Lagesensors 7 oder 9. Aus der bekannten Geometrie des Instrumentes 1 kann im Idealfall bereits aus den Lageangaben eines einzelnen Lagesensors die Lage des Instrumentenarbeitspunktes 11, also Ursprung und Orientierung des Koordinatensystems 12, berechnet werden.

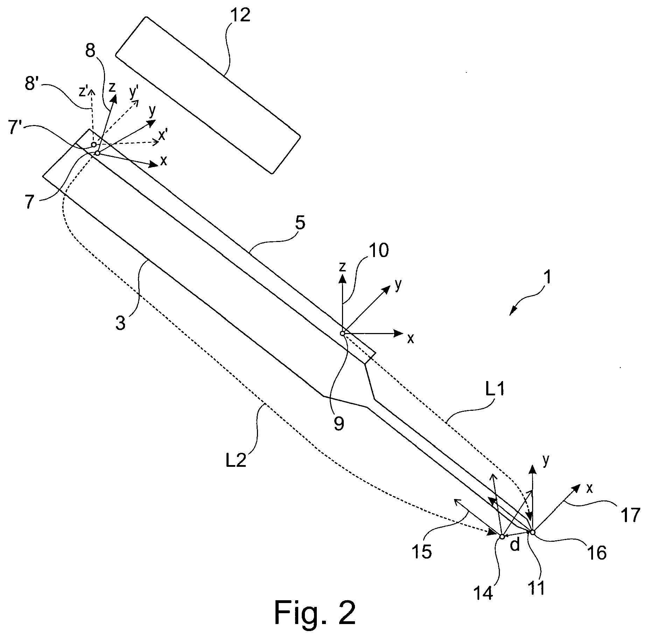

Fig. 2 zeigt das Instrument aus Fig. 1, wobei die gleichen Bezugszeichen gleiche Komponenten bedeuten. In dem in Fig. 2 dargestellten Fall befindet sich ein störendes Objekt 12 in der Nähe des Instrumentes 1. Das Objekt 12 ist in der Weise störend, dass es in der Lage ist, die durch die nicht gezeigte Feldspule oder Feldspulen erzeugten Felder derart zu verändern, dass die aus den Lagesensoren 7 und 9 ausgelesenen Signale in unerwünschter Weise ausgewertet werden. Dies führt dazu, dass die Auswertung der Lagemessdaten des Lagesensors 9, welcher einen großen Abstand zum Störobjekt 12 aufweist, in der Steuereinheit als ein Ergebnis für die Lage des Instrumentenarbeitspunktes ein Koordinatensystem 17 mit einem Ursprung 16 ermittelt, während die Auswertung der Lagemessdaten des Lagesensors 7 als Ergebnis ein Koordinatensystem 15 mit einem Ursprung 14 liefert. Die Abweichung zwischen den Ursprüngen 16 und 14 der Koordinatensysteme 17 und 15 wird als d bezeichnet. Der Betrag dieses Vektors d wird als Plausibilitätswert bezeichnet und wird im Folgenden weiter verwendet.

Der Steuereinheit der Auswertelogik liegt keine Information vor, welcher der Lagesensoren stärker gestört wird. Daher ist die Steuereinheit nicht dazu in der Lage, zu entscheiden, welches der Koordinatensysteme 15 mit Ursprung 14 oder 17 mit Ursprung 16 den Instrumentenarbeitspunkt besser darstellt. Zur Darstellung auf einer Anzeigevorrichtung wird daher die Instrumentenspitze mit einem Kreis einer Kugel versehen, deren Durchmesser dem Plausibilitätswert entspricht, so dass ein Benutzer eines derartigen Systems in der Lage ist, auf der Grundlage dieser Bildschirmdarstellung zu entscheiden, wie genau oder ungenau er im Augenblick gerade das Instrument im Raume anordnet.

Ein weiteres Problem ist, dass, wenn das Instrument bewegt wird, also ein dynamischer Zustand vorliegt, aufgrund der Messrate des Systems der Plausibilitätswert ebenfalls steigt. Das heißt, dass die Bewegung ebenfalls als Störung wahrgenommen wird, was als Eigenstörung bezeichnet wird. Da mit einem zu großen Plausibilitätswert behaftete Lagemessdaten nicht einer Visualisierung zugeführt werden, kann eine rasche Bewegung des Instrumentes zur Folge haben, dass die Navigationsinformation für das bewegte Instrument nicht angezeigt wird. Dies ist unkomfortabel für den Anwender. Ein durch die Plausibilitätsprüfung erlangter Sicherheitsgewinn geht somit zu Lasten der Verfügbarkeit der Navigationsinformation. Diese Einbuße muss vermieden werden, um das System ergonomisch einsetzen zu können.

Da die Störung zu einem unbestimmten Zeitpunkt und mit nicht zuvor bekannter Auswirkung auf die gemessene Instrumentenposition auftreten kann, werden folgende Anforderungen an eine Risiko minimierende Fehlerbehandlung gestellt:

Die Störungen, die die Messung der Instrumentenposition nach statischer Fehlerkorrektur aktuell beeinflussen, müssen vom System erkannt und quantifiziert werden.

Die Fehlererkennung untersucht in jedem Messzyklus die Messwerte zur Laufzeit.

Die Fehlererkennung soll nicht durch ein Referenzsystem optischer oder mechanischer Art unterstützt werden, weil dadurch der Systemaufbau unergonomisch und kostenintensiv wird.

Die erkannte quantifizierte Störung soll hinreichend genau mit dem tatsächlich auftretenden Positionsmessfehler an dem Instrumentenarbeitspunkt korrelieren. Hinreichend genau bedeutet hier, dass dynamische Positionsmessfehler, die über einem Sicherheitswert von maximal 2 mm liegen, mit einer Wahrscheinlichkeit von 95 % erkannt werden.

Messwerte, die mit einem dynamischen Positionsmessfehler beaufschlagt sind, werden weiter verarbeitet. Die Weiterverarbeitung führt dazu, dass die Messwerte korrigiert werden oder für Navigation ausgeschlossen oder speziell gekennzeichnet zur Anzeige gebracht werden.

Zur Erfüllung dieser Anforderungen wird ein Verfahren genutzt, das auf einer dynamischen Fehlererkennung basiert. Diese Fehlererkennung, im Folgenden auch als Plausibilitätsprüfung bezeichnet, wird nachfolgend näher beschrieben. Die hierauf aufbauenden weiteren Komponenten werden anschließend erläutert.

Es ist vorgesehen, zwischen einer Eigenstörung und einer Fremdstörung, hervorgerufen durch ein weiteres Objekt, zu unterscheiden und diese Störklassen unterschiedlich zu behandeln. Die Klasse Fremdstörung wird aus Gründen der Risikominimierung wie oben beschrieben behandelt. Das bedeutet, dass zumindest eine Fehlerkugel um den Instrumentenarbeitspunkt herum dargestellt wird. Die Klasse der Eigenstörung wird nochmals in Bewegungszustände unterteilt. Während der Anwendung des navigierten Instrumentes treten unterschiedliche Bewegungszustände auf. Mit jedem Bewegungszustand ist eine Genauigkeitsanforderung an die dargestellte Position verknüpft.

Die Tabelle 1 zeigt die definierten Zustandsklassen und die Tabelle 2 beschreibt die Anforderungen an die einzelnen Bewegungszustände qualitativ. Aus der jedem Bewegungszustand zugeordneten Genauigkeitsanforderung folgt, dass Störungen bei dynamischen Vorgängen, wie das Bewegen des Instrumentes im Arbeitsraum, weniger strikt behandelt werden müssen. Es heißt, dass der Schwellwert für die Risikoklasse bei dem Zustand Bewegen höher liegen kann. Somit werden diese Eigenstörungen nicht herausgefiltert.

**Tabelle 1**

| **Zustand** | **Zustandsbeschreibung** | **Beschreibung der Messsituation** |
|---|---|---|
| z = 1 | Fremdstörung | Die gemessene Instrumentenposition wirddurch ein weiteres Objekt beeinflusst |
| z = 2 | Eigenstörung - Positionieren des Instruments | Die gemessene Instrumentenposition wird nicht oder durch unzureichende Kalibrierdaten beeinflusst. |
| z = 3 | Eigenstörung - Rotieren des Instruments | Die gemessene Instrumentenposition durch die Rotationsbewegung beeinflusst. |
| z = 4 | Eigenstörung - Bewegen des Instruments | Die gemessene Instrumentenposition durch die Translationsbewegung beeinflusst. |

**Tabelle 2**

| **Bewegungszustand** | **Definition** | **Genauigkeitsanforderung** |
|---|---|---|
| Positionieren | Das Instrument wird an einer Position still gehalten oder mit geringer Geschwindigkeit (< 5 mm/s) bewegt oder rotiert. | Hoch - Der Anwender gleicht in diesem Zustand, die aktuelle Position des Instruments am Patienten mit der dargestellten Position am Bildschirm ab. Die Information bedarf einer geringen Messunsicherheit, da u. U. Abstände zu sensiblen Strukturen verdeutlicht werden sollen und das Instrument an dieser Position eine Arbeit verrichtet (z.B. Bohren). Das Instrument wird aktiv eingesetzt. |
| Rotieren | Das Instrument wird geschwenkt oder um die eigene Achse gedreht. | Mittel - Der Anwender richtet das Instrument aus, um den Bewegungszustand *Positionieren* zu erreichen. Das Instrument wird in diesem Zustand noch nicht aktiv eingesetzt. |
| Bewegen | Das Instrument wird mit einer Geschwindigkeit > 5 mm/s bewegt (Translation). | Niedrig - Der Anwender bewegt das Instrument im Arbeitsraum. Durch die Darstellung am Bildschirm erhält er eine grobe Orientierung in der Anatomie. Aufgrund der Bewegung ist ein genauer visueller Abgleich mit den Bilddaten nicht möglich. In diesem Bewegungszustand wird der Eingriffsort lokalisiert. |

Somit ist ein Verfahren zum Erzeugen von Bilddaten für eine Darstellung eines Instrumentes, insbesondere eines medizinischen Instrumentes, auf einer Anzeigevorrichtung geschaffen, wobei das Verfahren die folgenden Schritte umfasst: Erfassen von Lagemessdaten von wenigstens einem an dem Instrument angeordneten Lagesensor, Bestimmen eines Bewegungszustandes des Instruments auf der Grundlage der erfassten Lagemessdaten, Filtern der erfassten Lagemessdaten auf der Grundlage des bestimmten Bewegungszustandes und Bereitstellen eines Bilddatensatzes für eine Darstellung des Instruments auf der Anzeigevorrichtung wenigstens auf der Grundlage der gefilterten Lagemessdaten, welcher konfiguriert ist, das Instrument auf der Anzeigevorrichtung darzustellen.

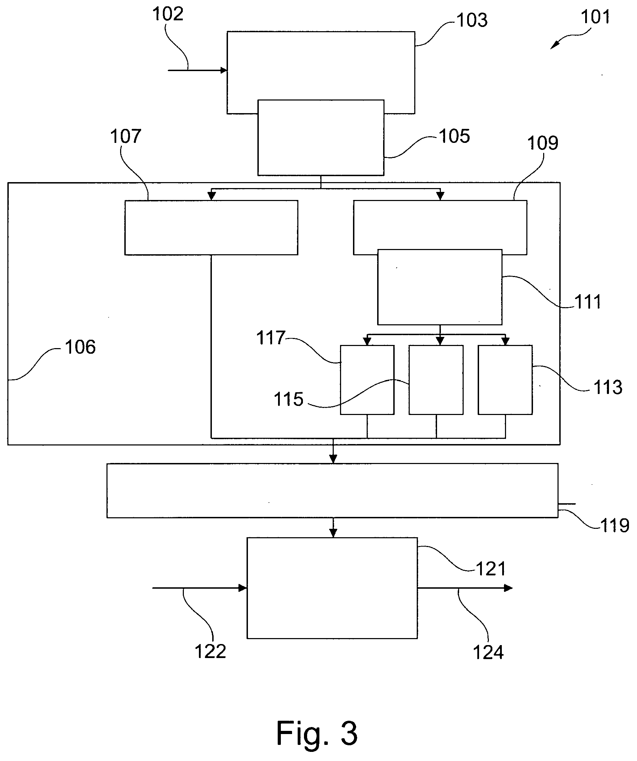

Fig. 3 veranschaulicht ein Konzept einer Umsetzung einer adaptiven Schwellwertvorgabe. Demgemäß ist zunächst ein Signalspeicher 103 vorgesehen, der benötigt wird, um einen Signalverlauf für eine nachfolgende statistische Analyse vorzuhalten. Es werden einem Eingang 102 zugeführte aktuelle Messwerte beispielsweise Positions- oder Lagedaten, p(k) sowie n vorherige Messwerte gespeichert. Ferner wird dem Eingang der aktuelle Plausibilitätswert d(k) zugeführt.

Ein Störungsklassifikator 105 schließt aus den gespeicherten Signalen auf die Art der Störung. Es wird eine Unterscheidung zwischen Fremdstörung und Eigenstörung getroffen. Keine erkennbaren Störungen werden ebenfalls als Eigenstörungen klassifiziert.

Somit wird bereits am Eingang einer Zustandsbestimmung 106 eine Trennung zwischen einer Fremdstörungsbehandlung 107 und einer Eigenstörungsbehandlung 109 vorgenommen.

Wurde durch den Störungsklassifikator 105 bestimmt, dass eine Eigenstörung vorliegt, so schließt ein Bewegungsklassifikator 111 aus dem Signal auf den aktuellen Bewegungszustand des Instrumentes. Dafür wurden zuvor die Signalverläufe für die einzelnen Zustände analysiert und Kriterien für die Klassifizierung herausgearbeitet, wie weiter unten gezeigt wird. Mit Hilfe dieser Kriterien kann die Klassifizierung zur Laufzeit vorgenommen werden. Es sind die Bewegungszustände Positionieren 117, Bewegen 115 und Rotieren 113 definiert. In Tabelle 2 sind diese Zustände genauer erläutert.

Der in der Zustandsermittlung 106 ermittelte Bewegungszustand, welcher die aktuelle Messsituation beschreibt, wird einer Schwellwertparametrisierung s = f(z) zugeführt, 119. Die Schwellwertparametrisierung s = f(z), 119, ordnet jedem ermittelten Bewegungszustand einen Schwellwert zu, welcher in einer nachfolgenden Stufe ausgewertet wird. Geeignete Schwellwerte wurden zuvor experimentell ermittelt. Der von der Schwellwertparametrisierung 119 ermittelte Schwellwert wird dem Schwellwertfilter 121 zugeführt. Der Schwellwertfilter 121 vergleicht den ihm über den Eingang 122 zugeführten aktuellen Plausibilitätswert p(k) mit dem parametrisierten Schwellwert s = f(z) für die Risikoklasse. Messwerte, die in die Risikoklasse fallen, werden gefiltert. Der Schwellwert wird fortlaufend an den aktuellen Bewegungszustand angepasst.

Der adaptive Schwellwertfilter erlaubt für den Fall, dass keine Fremdstörung erkannt wird, eine Anzeige der Navigationsinformation bei höherer Dynamik des Instrumentes. Gleichzeitig wird das Sicherheitskonzept nicht verletzt.

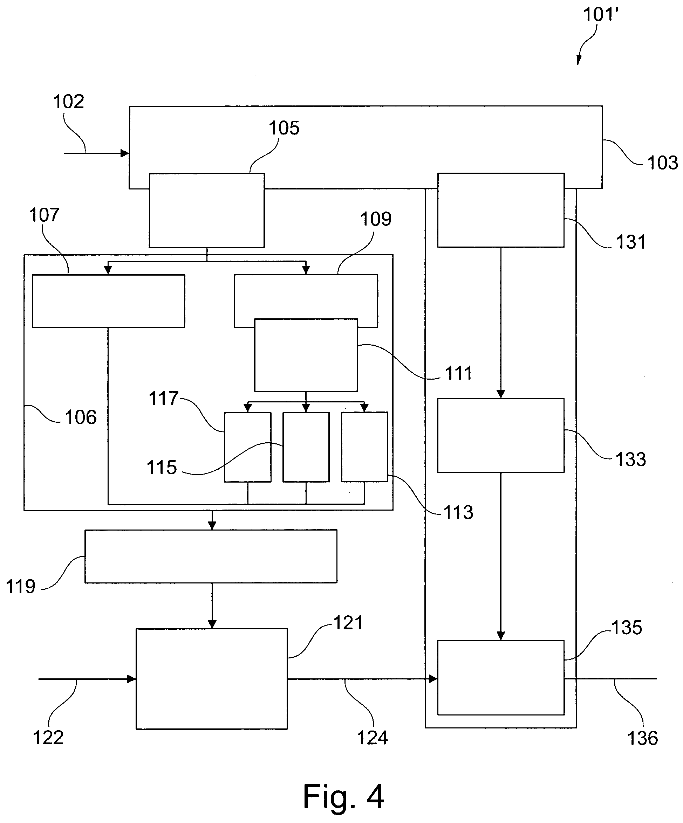

Die Fig. 4 veranschaulicht ein erweitertes Filterkonzept. Die oben beschriebene Behandlung dynamischer Fehler zielte ausschließlich auf das Herausfiltern, also das Verwerfen fehlerbehafteter Messwerte. Mit der Signalkonditionierung soll das Filterkonzept nochmals um eine Komponente erweitert werden, die dynamische Fehler, hervorgerufen durch Eigenstörung, korrigiert.

Das Filter 101' liefert zu jedem Messwert, also zu gemessenen Lage- und / oder Postitionsdaten p(k) am Eingang 112 einen korrigierten Messwert p'(k) am Ausgang 136.

Fig. 4 veranschaulicht die Funktionsweise des zu entwerfenden Filters. Die Blöcke 131, 133 und 135 gliedern sich in das bestehende Filterdesign zwischen dem Signalspeicher und dem eigentlichen Signalpfad ein. Die Vorverarbeitung kann parallel zu der Klassifizierung des Schwellwertfilters arbeiten. Die Komponente Signalkonditionierung 135, die die Positionsmessdaten manipuliert, wird im Signalpfad 124 hinter dem Schwellwertfilter 121 angeordnet. Die Datenrate im Signalpfad wird durch das Filterkonzept nicht verändert. Das heißt, dass die Verarbeitung der Signale in einem Messzyklus vorgenommen werden kann.

Die hinzugekommenen einzelnen Funktionsblöcke des erweiterten Filters sind eine Datenaufbereitung 131, welche die gespeicherten Signaldaten nutzt, um weitere Signale daraus zu generieren, beispielsweise Geschwindigkeit, Korrelation und / oder Varianz. In einem Modellvergleich 133 können die Signalverläufe analysiert und mit den Vorgaben eines Modells verglichen werden. Daraus leiten sich Parameter für die Komponente der Signalkonditionierung 135 ab. Die Signalkonditionierung 135 manipuliert die aktuell gemessenen und gefilterten Positionsmesswerte p(k). Der am Ausgang 136 anliegende Wert p'(k) ist somit eine Funktion von p(k) sowie zumindest des ermittelten Bewegungszustands.

Der Bewegungsklassifikator 111 bewertet die gespeicherten Signalverläufe hinsichtlich des Störungs- und Bewegungszustandes des Instrumentes. Dafür wurden zuvor in einer Untersuchung geeignete Kriterien für eine Klassifikation gefunden. In der Untersuchung wurden Signalverläufe der Instrumentenarbeitspunktverläufe in Abhängigkeit von den unterschiedlichen Lagesensordaten aufgezeichnet. Hierzu wurde jeweils eine bestimmte Bewegung, wie beispielsweise Translation, Rotation oder Positionieren durchgeführt. Die Signalverläufe wurden hinsichtlich eindeutiger Merkmale analysiert. Für die Signalanalyse wurden drei Größen eingeführt, mit denen die Signale charakterisiert werden können: Zunächst wurde der Korrelationskoeffizient eingeführt, welcher die Korrelation zwischen den beiden Instrumentenarbeitspunktsignalen für die gespeicherten Messwerte beschreibt. Als nächstes wurde die Memory-Varianz herangezogen, die ein Maß für die Streuung der gespeicherten Messwerte ist. Schließlich wurde eine Instant-Varianz herangezogen, welche ein Maß für die Streuung der letzten drei Messwerte ist. Dieses Signal folgt raschen Änderungen schneller als die Memory-Varianz. Die Varianz wurde eingeführt, weil bei einer Bewegung die Varianz höher ist als bei einem stillgehaltenem, also positioniertem, Instrument.

Es wurde festgestellt, dass eine Translation nicht vorliegt, wenn eines der folgenden Kriterien zutrifft: Kriterium 1: die Korrelationskoeffizienten aller Freiheitsgrade (x, y, z) sind kleiner als ein experimentell ermittelter Wert; Kriterium 2: die Korrelationskoeffizienten von 2 Freiheitsgraden sind negativ; oder Kriterium 3: die Instant-Varianzen aller Freiheitsgrade sind kleiner als ein experimentell gefundener Wert. Hinsichtlich einer Rotation, die eine Schwenkbewegung des Instrumentes beschreibt, bei welcher der Instrumentenarbeitspunkt auf einer Position verbleibt, wurde festgestellt, dass eine Rotierbewegung nicht vorliegt, wenn das Kriterium 4 zutrifft, dass die Instant-Varianzen aller Freiheitsgrade kleiner als ein experimentell bestimmter Wert sind.

Ein durch einen Fremdkörper hervorgerufener Störungszustand liegt vor, wenn eines der folgenden Kriterien erfüllt wird: Kriterium 5: Die Memory-Varianzen aller Freiheitsgrade sind kleiner als ein experimentell bestimmter Wert; oder Kriterium 6: Die Korrelationskoeffizienten von genau zwei Freiheitsgraden sind kleiner als ein experimentell bestimmter Wert und der verbleibende Freiheitsgrad hat einen Korrelationskoeffizienten der kleiner ist als ein negativer experimentell bestimmter Wert.

Die drei Größen Korrelationskoeffizient, Instant-Varianz und Memory-Varianz werden zu jedem Messzyklus k mittels des Bewegungsklassifikators 111 aus dem Signalspeicher für die Freiheitsgrade beider Positionsmessungen berechnet.

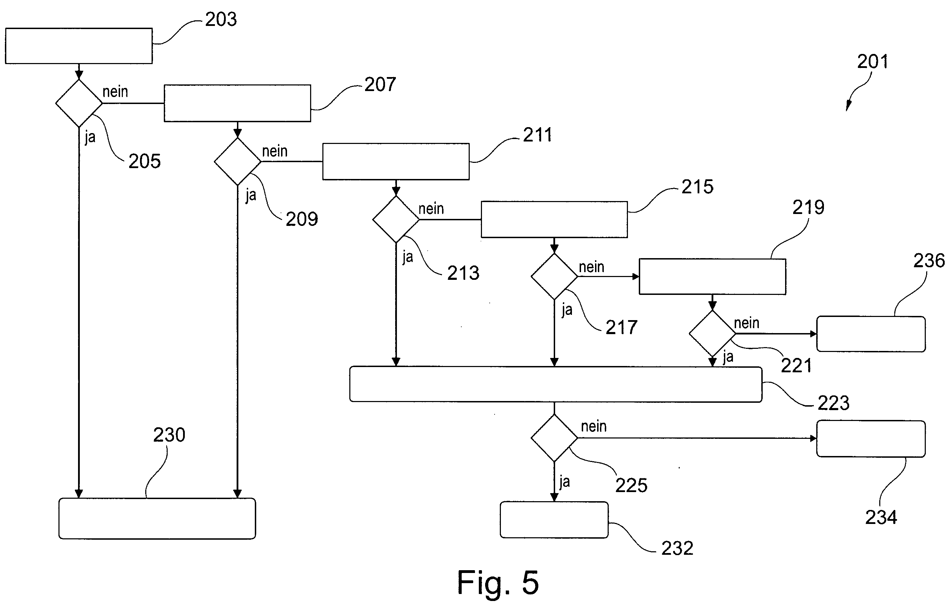

Die Fig. 5 veranschaulicht einen Algorithmus zur Klassifizierung, also zur Bestimmung eines Bewegungszustandes, auf der Grundlage der oben definierten Kriterien. Dabei werden die Kriterien sukzessiv geprüft und Entscheidungen getroffen. Als Ergebnis liefert der Algorithmus einen Bewegungszustand, der für die Parametrisierung des Schwellwertfilters herangezogen wird. Der in Fig. 5 dargestellte Klassifizierungsalgorithmus 201 prüft also die oben definierten einzelnen Kriterien und ermittelt schließlich einen Bewegungszustand des Instrumentes. In einem Schritt 203 überprüft der Algorithmus, ob das Kriterium 5 erfüllt ist, und verzweig in 205. In dem Falle, dass das Kriterium 5 nicht erfüllt ist, setzt der Algorithmus in 207 fort. In dem Falle, dass das Kriterium 5 erfüllt ist, endet der Algorithmus in 230 und bestimmt den Bewegungszustand als Störungszustand, z = 1.

In 207 überprüft der Algorithmus das Kriterium 6, und verzweigt in 209. In dem Falle, dass das Kriterium 6 nicht erfüllt ist, setzt der Algorithmus in 211 fort. In dem Falle, dass das Kriterium 6 erfüllt ist, endet der Algorithmus in 230 und bestimmt den Bewegungszustand als Störungszustand. In 211 prüft der Algorithmus das Kriterium 1, und verzweigt in 213. In dem Falle, dass das Kriterium 1 nicht erfüllt ist, setzt der Algorithmus in 215 fort. In dem Falle, dass das Kriterium 1 erfüllt ist, setzt der Algorithmus in 223 fort. In 215 prüft der Algorithmus das Kriterium 2, und verzweigt in 217. In dem Falle, dass das Kriterium 2 nicht erfüllt ist, setzt der Algorithmus in 219 fort. In dem Falle, dass das Kriterium 2 erfüllt ist, setzt der Algorithmus in 223 fort. In 219 überprüft der Algorithmus das Kriterium 3, und verzweigt in 221. In dem Falle, dass das Kriterium 3 nicht erfüllt ist, endet der Algorithmus in 236 und hat als Bewegungszustand die Translation, z = 4, bestimmt. In dem Falle, dass das Kriterium 3 erfüllt ist, setzt der Algorithmus in 223 fort. In 223 prüft der Algorithmus das Kriterium 4, und verzweigt in 225. In dem Falle, dass das Kriterium 4 nicht erfüllt ist, endet der Algorithmus in 234 und hat den Bewegungszustand als Rotieren bestimmt, z = 3. In dem Falle, dass das Kriterium 4 erfüllt ist, endet der Algorithmus in 232 und hat den Bewegungszustand als Positionieren bestimmt, z = 2.

Jedem der ermittelten Bewegungszustände, also Störungszustand, Positionieren, Rotieren und Translation, ist ein Schwellwert zugeordnet. Wie in den Fig. 3 und 4 veranschaulicht ist, entscheidet ein Schwellwertfilter, ob der aktuelle Plausibilitätswert d einen der parametrisierten Schwellwerte überschreitet oder unterschreitet. In dem Falle, dass der ermittelte Schwellwert überschritten wird, wird der zugehörige Messwert verworfen, das heißt er wird in 124 nicht ausgegeben und steht somit nicht für eine Darstellung des Instruments auf der Anzeigevorrichtung zur Verfügung. In dem Falle, dass der Plausibilitätswert kleiner ist als der parametrisierte Schwellwert aus 119 wird der aktuelle Positionswert in 121 nach 124 durchgelassen. Die Zuordnung eines Bewegungszustandes aus dem Bewegungsklassifikator zu einem Schwellwert wird als Parametrisierung bezeichnet, welche als eine Tabelle für jeden Bewegungszustand hinterlegt ist. Zur Erstellung der Tabelle wurden die einzelnen Schwellwerte experimentell für das Instrument ermittelt.

Die letzte Komponente im Signalpfad ist die Visualisierung der Positionsdaten, also eine Darstellung des Instrumentes auf einer Anzeigevorrichtung. Die korrigierten und gefilterten Daten werden in ein Bilddatenkoordinatensystem transformiert und angezeigt. Die Visualisierung umfasst folgende zusätzliche Eigenschaften: 1. Fehlerabschätzung: Die Messunsicherheit, die durch die Registrierung und Messstörungen besteht, wird abgeschätzt. Als Maß für die Registrierunsicherheit wird ein Gütewert aus dem Registrieralgorithmus verwendet. Der Wert gibt die maximale Abweichung der zueinander registrierten Punkte an. Dieser Wert wird vereinfacht richtungsunabhängig verwendet. Als Maß für fehlerbehaftete Positionsmesswerte dient der Plausibilitätswert d. Dieser wird in jedem Messzyklus k berechnet. 2. Visualisieren der Messunsicherheit: Die abgeschätzte Messunsicherheit wird als Raum um die dargestellte Position des Instrumentenarbeitspunktes in Form einer Kugel visualisiert. In orthogonalen Schnittansichten ist dies daher ein Kreis. Die Dynamik dieser Anzeige ermöglicht es einem Anwender, den Störungszustand der aktuellen Positionsmessung zu beurteilen. 3. Visualisierung des Störungszustandes: Messdaten, die durch den Schwellwertfilter verworfen wurden, werden nicht für die Navigationsanzeige verwendet. Dieser Zustand übermäßiger Störung wird dem Anwender durch eine rote Einfärbung des, in den Bilddaten repräsentierten, Instrumentes signalisiert. Das Instrument wird auf der letzten gültigen gemessenen Position dargestellt, bis ein neuer gültiger Messwert vorliegt.

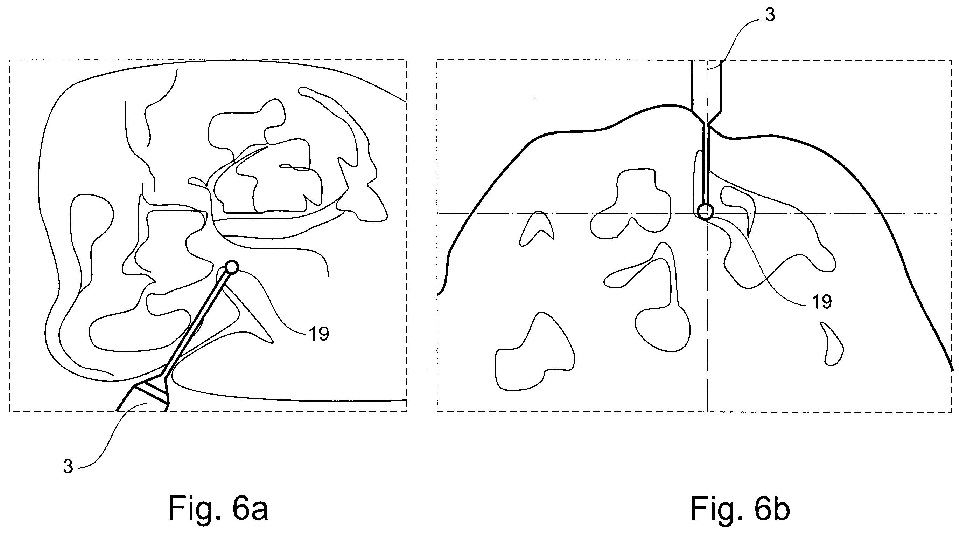

Die Fig. 6a veranschaulicht eine Darstellung eines Instrumentes an einem Gesicht eines Patienten, wobei eine aktuelle Messunsicherheit in Form eines Kreises 19 dargestellt ist. In der Fig. 6b ist dieselbe Situation in einem Schnitt in einer Draufsicht dargestellt.

Es wurde gezeigt, dass eine Signalkonditionierung das Positionsmesssignal zur Laufzeit auf der Basis eines vorgegebenen Modells korrigiert. Das Filter zielt dabei auf die Korrektur von dynamischen Fehlern, die durch die Eigenbewegung des Instrumentes, also dynamische Effekte, hervorgerufen werden. Oben wurde bereits der dynamische Effekt beim Rotieren des Instrumentes diskutiert. Ebenso tritt bei der Translation des Instrumentes ein dynamischer Effekt auf. Mit der Bewegungsgeschwindigkeit steigt das Messrauschen des Positionssignals. Dies ist durch die interne Signalvorverarbeitung im Messsystem bewirkt, die bei geringen Bewegungsgeschwindigkeiten eine gleitende Mittelwertbildung vornimmt, wodurch das Signal geglättet wird. Während der Bewegung wird die Mittelwertbildung ausgesetzt, um der erhöhten Dynamik gerecht zu werden. Das Signal rauscht jedoch in diesem Fall stärker.

Zur Filterung des Bewegungsrauschens sind zwei alternative Verfahren möglich, das Kalman Filter und das Varianzfilter. Das Kalman-Filter ist eine verbreitete Methode zur Abschätzung von Bewegungen aus verrauschten Signalen. Vereinfacht gesagt vergleicht das Kalman-Filter die aktuellen Bewegungsvariablen, das sind Ort, Geschwindigkeit und Beschleunigung, mit dem Bewegungsmodell bzw. der Bewegungsgleichung, das bzw. die im Filter hinterlegt ist. Das Kalman-Filter wurde für die kritische Bewegung der Rotation des Instrumentes auf einem Punkt untersucht. An diesen Bewegungszustand ist eine höhere Genauigkeitsanforderung gestellt, als an den Bewegungszustand der Translation. Es hat sich herausgestellt, dass das Kalman-Filter für den Bewegungszustand der Rotation nicht sinnvoll einsetzbar ist.

Es hat sich herausgestellt, dass ein Varianzfilter, das die bereits erläuterten Werte der Memory-Varianz und der Instant-Varianz zur Signalklassifizierung auf der Grundlage des Signalspeicherinhaltes verwendet, geeigneter sind als Kalman-Filter. Durch die ermittelten experimentellen Parameter wird eine gute Qualität des Filters für Rotationen um die Instrumentenachse erreicht, so dass dem Benutzer eine komfortable Bedienung eines wie oben beschrieben aufgebauten Systems ermöglicht ist.

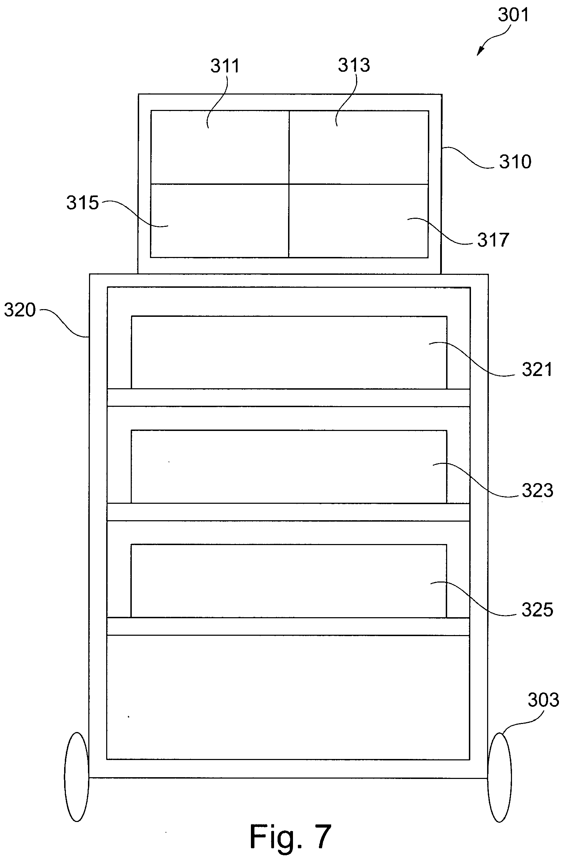

In der Fig. 7 ist ein Endoskopiesystem 301 veranschaulicht, das unabhängig von dem oben beschriebenen Verfahren, System, Computerprogrammprodukt, Computerprogramm und Datenträger bereitgestellt ist, aber in der Lageg ist, mit dem oben beschriebenen Verfahren, System, Computerprogrammprodukt, Computerprogramm und Datenträger wirksam zusammenzuarbeiten. Das Endoskopiesystem 301 umfasst wenigstens einen Monitor 310 und wenigstens einen Navigationsmodul 321 und ist mittels Rollen oder Rädern 303 bequem transportierbar. Das Endoskopiesystem 301 ist dazu konfiguriert, wenigstens eine synchronisierte Video- und Navigationsinformation auf dem Monitor, beispielsweise einem Endoskopiemonitor, bereitzustellen. Beispielsweise ist das Endoskopiesystem 301 dazu konfiguriert, eine auf der Grundlage des oben beschriebenen Verfahrens synchronisierte Video- und Navigationsinformation auf dem Monitor 310 darzustellen. Die Synchronisation umfasst beispielsweise eine Aktualisierung der, wie oben beschrieben, um den Instrumentenarbeitspunkt herum dargestellten, auf der Grundlage des Plausibilitätswertes und der erfassten Lagemessdaten bestimmten Kugel zur Veranschaulichung einer Messunsicherheit. Die Aktualisierung der Darstellung kann beispielsweise auf einer Aktualisierung des jeweils zur Anzeige gebrachten Bilddatensatzes beruhen. Vorteilhaft kann für eine gleichzeitige Visualisierung mehrerer Ansichten vorgesehen sein, den Monitor 310, im Betrieb, mittels einer geeigneten Ansteuerung des Monitors, in vier Teilansichten 311, 313, 315 und 317 zu unterteilen. Die Bezugszeichen 323 und 325 bezeichnen weitere am Endoskopieturm vorgesehene Standardmodule.

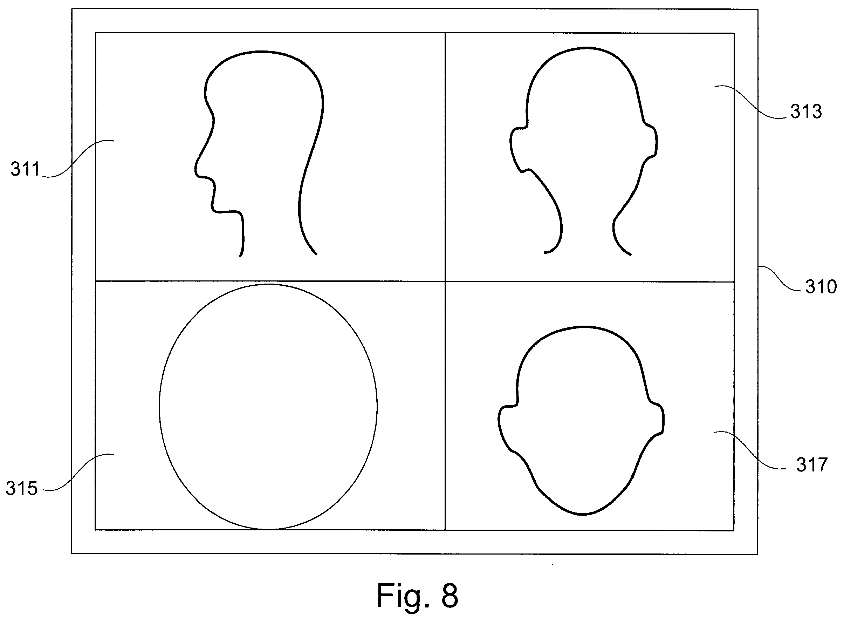

Die Fig. 8 stellt eine beispielhafte Darstellung von Endoskopieinformationen auf einem Endoskopiemonitor 310 dar. Der beispielsweise mittels geeigneter Monitoransteuerung in vier Teilansichten aufgeteilte Monitor 310 stellt in einer Teilansicht 311 einen sagittalen, in einer Teilansicht 313 einen coronalen und in Teilansicht 317 einen axialen Schnitt durch einen Kopf eines Patienten dar. In einer Teilansicht 315 können beispielsweise synchronisierte Video- und / oder Navigationsinformationen dargestellt werden. Diese Informationen können beispielsweise nach dem oben beschriebenen Verfahren berechnet und / oder aufbereitet worden sein.

Mit anderen Worten ist mittels des beschriebenen Endoskopiesystems 301 ein Dokumentations- und Behandlungsmodul für eine Navigation in der Medizin bereitgestellt. Das System zur Dokumentation und Durchführung von navigierten Eingriffen besteht zumindest aus einem Navigationsmodul 321, das als zusätzliche Komponente eines herkömmlichen Videoendoskopieturms bereitgestellt sein kann. Das System umfasst vorzugsweise wenigstens einen Monitor. Vorzugsweise ist dieses System nicht als zusätzliches mobiles oder stationäres System konfiguriert, sondern in dem Videoendoskopieturm integriert. Die aufgenommenen Videodaten können zusammen mit Navigationsdaten, die beispielsweise wie oben beschrieben synchronisiert sind, zu Dokumentationszwecken aufgezeichnet werden. Ferner umfasst das Modul vorzugsweise Schnittstellen für optische und / oder magnetische Navigationssensoren. Vorteilhaft ist es bei diesem System während einer Bedienung und / oder während eines Arbeitens mit dem System nicht erforderlich, Eingaben an dem System vorzunehmen. Dadurch ist eine Bedienperson entlastet und kann ihre Aufmerksamkeit vollständig auf die Arbeit an einer Umgebung des Instrumentenarbeitspunktes richten. Wenigstens ein elektromagnetischer Navigationssensor kann in einer festgelegten Ausrichtung an einem Operationstisch angebracht werden und mit dem Navigationsmodul 321 derart verbunden werden, dass die Sensordaten des elektromagnetischen Navigationssensors vom Navigationsmodul 321 ausgelesen und ausgewertet werden können und anschließend auf dem Monitor dargestellt werden können.

Wenigstens ein optischer Navigationssensor kann an einem selbsthaltenden Kugelgelenk befestigt und mit einer Hand einer Bedienperson ausgerichtet werden. Der optische Navigationssensor kann beispielsweise als Kamera ausgeführt sein. Der optische Navigationssensor kann derart mit dem Navigationsmodul 321 verbunden sein, dass die Sensordaten des elektromagnetischen Navigationssensors vom Navigationsmodul 321 ausgelesen und ausgewertet werden können und anschließend auf dem Monitor dargestellt werden können. Vorzugsweise ist das System derart konfiguriert, dass die Navigationsinformationen und die Videoinformationen auf zwei Monitore aufgeteilt und dargestellt werden können. Es kann beispielsweise vorgesehen sein, dass das Navigationsmodul über eine drahtlose Verbindung mit dem Navigationssensor kommuniziert. Eine derartige Verbindung könnte beispielsweise über ein Bluetooth-Protokoll bereitgestellt sein. Es kann ferner vorgesehen sein, dass das Navigationsmodul Patientenbilddaten über eine drahtlose Verbindung bezieht. Beispielsweise könnte über ein WLAN im Gebäude ein Zugriff auf eine Patientendatenbank eines zentralen Servers erfolgen.

Das vorgestellte System kann in geeigneter Ausführung einen oder mehrere der folgenden Vorteile bereitstellen: Das Navigationssystem kann als ein Modul für einen Endoskopieturm bereitgestellt sein, so dass eine Handhabbarkeit verbessert ist. Das Navigationssystem kann vollständig in dem modularen Endoskopieturm integriert sein. Ferner kann es kompatible Schnittstellen zu weiteren gängigen Endoskopiemoduln bereitstellen. Ein klinischer Arbeitsablauf wird deutlich verbessert, da kein zusätzliches Gerät erforderlich ist.

Der optische Navigationssensor, der beispielsweise als Kamera ausgeführt ist, ist separat vom Endoskopieturm auf einem Stativ positionierbar. Die Kameraschnittstelle ist derart konfiguriert, dass sie alle gängigen Navigationskameras unterstützt. Das Navigationssystem ist derart konfiguriert, dass es eine Videoeinblendung vornehmen kann, also, dass verschiedene bestimmte Bilddaten beispielsweise auf einem Bildschirmbereich in überlagerter Weise dargestellt werden, so dass es einem Bediener beispielsweise möglich ist, reale Bilddaten und bewertete und ausgewertete Sensordaten gleichzeitig und mit räumlicher Zuordnung zu erfassen und auf diese Weise während der Arbeit eine verbesserte Navigation des Instruments vornehmen zu können. Schließlich ist das beschriebene System in der Lage, Videodaten und Schichtbilddaten zu dokumentieren, also beispielsweise die Daten als momentane Standbilddaten oder als Datenströme mit einem Zeitstempel zu versehen und zu speichern.

## Patentansprüche

1. Verfahren zum Erzeugen von Bilddaten für eine Darstellung eines Instruments auf einer Anzeigevorrichtung, wobei das Verfahren die folgenden Schritte umfasst:
- Erfassen von Lagemessdaten von wenigstens zwei an dem Instrument angeordneten Lagesensoren,
- Bestimmen eines Bewegungszustandes des Instruments auf der Grundlage der erfassten Lagemessdaten,
- Filtern der erfassten Lagemessdaten auf der Grundlage des bestimmten Bewegungszustandes und
- Bereitstellen eines Bilddatensatzes für eine Darstellung des Instruments auf der Anzeigevorrichtung wenigstens auf der Grundlage der gefilterten Lagemessdaten, welcher konfiguriert ist, das Instrument auf der Anzeigevorrichtung darzustellen,
- wobei jeder der an dem Instrument angeordneten Lagesensoren konfiguriert ist, Lagemessdaten auszugeben, aus denen durch eine Datenverarbeitungseinrichtung jeweils ein Instrumentenarbeitspunkt bestimmt wird,
wobei der Betrag des Abstandes zwischen den beiden ermittelten Instrumentenarbeitspunkten als Plausibilitätswert bezeichnet wird, und
- durch die Datenverarbeitungseinrichtung zur Darstellung auf der Anzeigevorrichtung der Instrumentenarbeitspunkt mit einem Kreis einer Kugel versehen wird, deren Radius dem Plausibilitätswert entspricht.

2. Verfahren nach Anspruch 1, **dadurch gekennzeichnet, dass** die erfassten Lagemessdaten auf Grundlage wenigstens einer aktuellen Lagemessung mit aktuell erfassten Lagemessdaten gebildet werden.

3. Verfahren nach Anspruch 1 oder 2, **dadurch gekennzeichnet, dass** die Lagemessdaten mittels mehrerer Lagemessungen erfasst werden.

4. Verfahren nach mindestens einem der vorangehenden Ansprüche, soweit auf Anspruch 2 rückbezogen, **dadurch gekennzeichnet, dass** der Bewegungszustand des Instruments auf der Grundlage der aktuell erfassten Lagemessdaten und in der Vergangenheit erfasster Lagemessdaten bestimmt wird.

5. Verfahren nach mindestens einem der vorangehenden Ansprüche, dadurch **gekenn**z e i c h n e t, dass jedem Bewegungszustand ein Plausibilitätsschwellwert zugeordnet wird.

6. Verfahren nach Anspruch 1 und Anspruch 5, **dadurch gekennzeichnet, dass** das Filtern der erfassten Lagemessdaten einen Schritt zum Vergleichen des Plausibilitätswertes und des Plausibilitätsschwellwertes umfasst.

7. Verfahren nach Anspruch 6, **dadurch gekennzeichnet, dass** das Filtern der erfassten Lagemessdaten einen Schritt zum Akzeptieren der erfassten Lagemessdaten für den Fall, dass der Plausibilitätswert kleiner als der oder gleich dem Plausibilitätsschwellwert ist, und einen Schritt zum Verwerfen der erfassten Lagemessdaten für den Fall umfasst, dass der Plausibilitätswert größer als der Plausibilitätsschwellwert ist.

8. Verfahren nach Anspruch 1 oder 2, **dadurch gekennzeichnet, dass** der Bilddatensatz wenigstens auf Grundlage der aktuell erfassten Lagemessdaten und des Plausibilitätswertes gebildet wird.

9. Verfahren nach mindestens einem der vorangehenden Ansprüche, soweit auf Anspruch 4 rückbezogen, **dadurch gekennzeichnet, dass** auf der Grundlage der erfassten Lagemessdaten und des Plausibilitätswertes bestimmt wird, ob eine Eigenstörung und / oder eine Fremdstörung vorliegt.

10. Verfahren nach Anspruch 9, **dadurch gekennzeichnet, dass** die Fremdstörung auf der Grundlage des Plausibilitätswertes und die Eigenstörung auf der Grundlage der erfassten Lagemessdaten bestimmt werden.

11. System zum Darstellen eines Instruments (1) mit:
- wenigstens zwei an dem Instrument (1) angeordneten Lagesensoren (7, 9), die konfiguriert sind, Lagemessdaten für das Instrument (1) zu erfassen,
- einer Anzeigevorrichtung (310) und
- einer Datenverarbeitungseinrichtung, die mit den wenigstens zwei Lagesensoren (7,9) und der Anzeigevorrichtung (310) verbunden und konfiguriert ist, einen Bilddatensatz für eine Darstellung des Instruments (1) auf der Anzeigevorrichtung (310) zu erzeugen,
- wobei jeder der an dem Instrument (1) angeordneten Lagesensoren (7, 9) konfiguriert ist, Lagemessdaten auszugeben, aus denen durch die Datenverarbeitungseinrichtung jeweils ein Instrumentenarbeitspunkt bestimmbar ist, wobei der Betrag des Abstandes zwischen den beiden ermittelten Instrumentenarbeitspunkten als Plausibilitätswert bezeichnet wird, und
- die Datenverarbeitungseinrichtung weiterhin so eingerichtet ist, dass zur Darstellung auf der Anzeigevorrichtung (310) der Instrumentenarbeitspunkt mit einem Kreis einer Kugel versehen wird, deren Durchmesser dem Plausibilitätswert entspricht.

12. System nach Anspruch 11, **dadurch gekennzeichnet, dass** an dem Instrument mehrere Lagesensoren angeordnet sind, wobei wenigstens ein Lagesensor an einem distalen Ende und wenigstens ein anderer Lagesensor an einem proximalen Ende des Instruments angeordnet sind.

13. System nach Anspruch 11 oder 12, **dadurch gekennzeichnet, dass** eine Feldspule zum Erzeugen eines Feldes vorgesehen ist und die wenigstens zwei Lagesensoren als elektromagnetische Lagesensoren konfiguriert sind, welche auf das Feld ansprechen.

14. Computerprogramm-Produkt, das Softwaremittel zur Durchführung eines Verfahrens nach einem der Ansprüche 1 bis 10 aufweist, wenn das Verfahren automatisiert in einem System nach einem der Ansprüche 11 bis 13 zum Darstellen eines Instruments ausgeführt wird.

## Claims

1. A method for producing picture data for a representation of an instrument on a display device, wherein the method comprises the following steps:
- detecting position measurement data by at least two position sensors arranged on the instrument,
- determining a movement condition of the instrument on the basis of the acquired position measurement data,
- filtering the acquired position measurement data on the basis of the determined movement condition and
- providing a picture data set for a representation of the instrument on the display device, at least on the basis of the filtered position measurement data, said picture data set being configured to represent the instrument on the display device,
- wherein each of the position sensors which are arranged on the instrument is configured to output position measurement data, from which data an instrument operating point is determined in each case by way of a data processing device, wherein the magnitude of the distance between the two determined instrument operating points is indicated as a plausibility value, and
- the instrument operating point is provided with a circle of a ball by way of the data processing device for representation on the display device, the radius of said circle corresponding to the plausibility value.

2. Method according to claim 1, **characterised in that** the detected position measurement data is formed on the basis of at least one current position measurement with currently acquired position measurement data.

3. Method according to claim 1 or 2, **characterised in that** the position measurement data is acquired by way of several position measurements.

4. Method according to at least one of the preceding claims, inasmuch as referring back to claim 2, **characterised in that** the movement condition of the instrument is determined on the basis of the currently acquired position measurement data and position measurement data acquired in the past.

5. Method according to one of the preceding claims, **characterised in that** a plausibility threshold value is assigned to each movement condition.

6. Method according to claim 1 and claim 5, **characterised in that** the filtering of the acquired position measurement data comprises a step for comparing the plausibility value and the plausibility threshold value.

7. Method according to claim 6, **characterised in that** the filtering of the acquired position measurement data comprises a step for accepting the acquired position measurement data in the case that the plausibility value is smaller than or equal to the plausibility threshold value, and comprises a step for rejecting the acquired position measurement data in the case that the plausibility value is larger than the plausibility threshold value.

8. Method according to claim 1 or 2, **characterised in that** the picture data set is formed at least on the basis of the currently acquired position measurement data and of the plausibility value.

9. Method according to one of the preceding claims, inasmuch as referring back to claim 4, **characterised in that** it is determined as to whether an internal disturbance and/or an external disturbance is present on the basis of the acquired position measurement data and the plausibility value.

10. Method according to claim 9, **characterised in that** the external disturbance is determined on the basis of the plausibility value and the internal disturbance on the basis of the acquired position measurement data.

11. A system for representing an instrument (1) with:
- at least two position sensors (7, 9) which are arranged on the instrument (1) and are configured to detect position measurement data for the instrument (1),
- a display device (310) and
- a data processing device which is connected to the at least two position sensors (7, 9) and the display device (310), and is configured to produce a picture data set for a representation of the instrument (1) on the display device (310),
- wherein each of the position sensors (7, 9) which are arranged on the instrument (1) is configured to output position measurement data, from which an instrument operating point can be determined in each case by way of a data processing device, wherein the magnitude of the distance between the two determined instrument operating points is indicated as a plausibility value, and
- the data processing device is moreover configured to provide the instrument operating point with a circle of a ball for representation on the display device (310), the radius of said circle corresponding to the plausibility value.

12. System according to claim 11, **characterised in that** several position sensors are arranged on the instrument, wherein at least one position sensor is arranged on a distal end of the instrument and at least another position sensor is arranged on a proximal end of the instrument.

13. System according to claim 11 or 12, **characterised in that** a field coil is provided for producing a field, and the at least two position sensors are configured as electromagnetic position sensors which respond to the field.

14. A computer program product which comprises software means for carrying out a method according to one of the claims 1 to 10, when the method is carried out in an automated manner in a system according to one of the claims 11 to 13 for representing an instrument.

## Revendications

1. Procédé de génération de données d'images pour une représentation d'un instrument sur un dispositif d'affichage, le procédé comprenant les étapes suivantes :
- relevé de données de mesure de position d'au moins deux capteurs de position disposées sur l'instrument,
- détermination d'un état de mouvement de l'instrument sur la base des données de mesure de position relevées,
- filtrage des données de mesure de position relevées sur la base de l'état de mouvement déterminé et
- mise à disposition d'un jeu de données d'images pour une représentation de l'instrument sur le dispositif d'affichage au moins sur la base des données de mesure de position filtrées, qui est conçu pour représenter l'instrument sur le dispositif d'affichage,
- chacun des capteurs de position disposés sur l'instrument étant conçu pour générer des données de mesure de position, à partir desquelles un point de travail de l'instrument est déterminé par un dispositif de traitement de données, la valeur de la distance entre les deux points de travail de l'instrument déterminés étant désignée comme une valeur de plausibilité et
- le point de travail de l'instrument est muni, par le dispositif de traitement de données, pour la représentation sur le dispositif d'affichage, d'un cercle d'une sphère dont le rayon correspond à la valeur de plausibilité.

2. Procédé selon la revendication 1, **caractérisé en ce que** les données de mesure de position relevées sont constituées, sur la base d'au moins une mesure de position actuelle, des données de mesure de position actuellement relevées.

3. Procédé selon la revendication 1 ou 2, **caractérisé en ce que** les données de mesure de position sont relevées au moyen de plusieurs mesures de position.

4. Procédé selon au moins l'une des revendications précédentes, avec référence à la revendication 2, **caractérisé en ce que** l'état de mouvement de l'instrument est déterminé sur la base des données de mesure de position actuellement relevées et des données de mesure de position relevées dans le passé.

5. Procédé selon au moins l'une des revendications précédentes, **caractérisé en ce qu'**à chaque état de mouvement correspond une valeur seuil de plausibilité.

6. Procédé selon la revendication 1 et la revendication 5, **caractérisé en ce que** le filtrage des données de mesure de position relevées comprend une étape de comparaison de la valeur de plausibilité et de la valeur seuil de plausibilité.

7. Procédé selon la revendication 6, **caractérisé en ce que** le filtrage des données de mesure de position relevées comprend une étape d'acceptation des données de mesure de position relevées pour le cas où la valeur de plausibilité est inférieure ou égale à la valeur seuil de plausibilité et une étape de rejet des données de mesure de position pour le cas où la valeur de plausibilité est supérieure à la valeur seuil de plausibilité.

8. Procédé selon la revendication 1 ou 2, **caractérisé en ce que** le jeu de données d'images est formé au moins sur la base des données de mesure de position actuellement relevées et de la valeur de plausibilité.

9. Procédé selon au moins l'une des revendications précédentes, avec référence à la revendication 4, **caractérisé en ce que**, sur la base des données de mesure de position relevées et de la valeur de plausibilité, il est déterminé si une perturbation interne et/ou une perturbation externe existe.

10. Procédé selon la revendication 9, **caractérisé en ce que** la perturbation externe est déterminée sur la base de la valeur de plausibilité et la perturbation interne est déterminée sur la base des données de mesure de position relevées.

11. Système pour la représentation d'un instrument (1), avec :
- au moins deux capteurs de position (7, 9) disposés sur l'instrument (1), qui sont conçus pour relever les données de mesure de position pour l'instrument (1),
- un dispositif d'affichage (310) et
- un dispositif de traitement de données qui est relié avec les au moins deux capteurs de position (7, 9) et le dispositif d'affichage (310) et conçu pour générer un jeu de données d'images pour une représentation de l'instrument (1) sur le dispositif d'affichage (310),
- chacun des capteurs de position (7, 9) disposés sur l'instrument (1) étant conçu pour générer des données de mesure de position, à partir desquelles un point de travail de l'instrument est déterminé par le dispositif de traitement de données, la valeur de la distance entre les deux points de travail de l'instrument déterminés étant désignée comme une valeur de plausibilité et
- le dispositif de traitement de données est en outre conçu de façon à ce que, pour la représentation sur le dispositif d'affichage (310), le point de travail de l'instrument est muni d'un cercle d'une sphère dont le diamètre correspond à la valeur de plausibilité.

12. Système selon la revendication 11, **caractérisé en ce que**, sur l'instrument, sont disposés plusieurs capteurs de position, au moins un capteur de position étant disposé au niveau d'une extrémité distale et au moins un autre capteur étant disposé au niveau d'une extrémité proximale de l'instrument.

13. Système selon la revendication 11 ou 12, **caractérisé en ce qu'**une bobine de champ est prévue pour la génération d'un champ et les au moins deux capteurs de position sont conçus comme des capteurs de position électromagnétiques, qui réagissent au champ.

14. Produit de programme informatique qui comprend des moyens logiciels pour la réalisation d'un procédé selon l'une des revendications 1 à 10 lorsque le procédé est effectué de manière automatisée dans un système selon l'une des revendications 11 à 13, pour la représentation d'un instrument.
